# EUROPÄISCHE PATENTSCHRIFT

(11) **EP 1 656 149 B1**
(45) Veröffentlichungstag und Bekanntmachung des Hinweises auf die Patenterteilung: **05.05.2010**
(21) Anmeldenummer: 04763831.7
(22) Anmeldetag: 05.08.2004
(51) Int. Cl.: A61K 35/74, C12N 1/21

(54) **VERSCHLIESSEN VON BAKTERIEN-GHOSTS DURCH BIOAFFINE WECHSELWIRKUNGEN**
SEALING OF BACTERIAL GHOSTS BY MEANS OF BIOAFFINITY INTERACTIONS
BLOCAGE DE BACTERIES FANTOMES PAR DES INTERACTIONS A AFFINITE BIOLOGIQUE

(30) Priorität: 05.08.2003 DE 10335796
(43) Veröffentlichungstag der Anmeldung: 17.05.2006
(73) Patentinhaber: Lubitz, Werner, Dr., 3420 Klosterneuburg/Kritzendorf (AT)
(72) Erfinder: Lubitz, Werner, Dr., 3420 Klosterneuburg/Kritzendorf (AT)
(74) Vertreter: Weiss, Wolfgang
(86) Internationale Anmeldenummer: PCT/EP2004/008790
(87) Internationale Veröffentlichungsnummer: WO 2005/011713

(56) Entgegenhaltungen:
- WO-A-03/053857
- DE-A- 19 909 770
- US-A1- 2003 003 511

## Beschreibung

Die Erfindung betrifft ein Verfahren zur Herstellung von verschlossenen Bakterienghosts über spezifische Wechselwirkungen zwischen Partnern eines bioaffinen Bindungspaares und die auf diese Weise erhältlichen Bakterienghosts. In die verschlossenen Bakterienghosts können Wirkstoffe verpackt werden. Die verschlossenen Ghosts können in der Medizin, im Agrarbereich und in der Biotechnologie eingesetzt werden.

Leere Bakterienhüllen, so genannte Bakterienghosts, können durch kontrollierte, heterologe Expression eines Gens, das eine partielle Lyse der Zellmembran bewirkt, in gram-negativen Bakterien hergestellt werden (EP-A-0 291 021). Ein Beispiel für ein derartiges lytisches Gen ist das Gen E des Bakteriophagen PhiX174, das für ein Polypeptid kodiert, welches in den Zellwandkomplex gram-negativer Bakterien inseriert und durch die Oligomerisierung zur Ausbildung einer transmembranen Tunnelstruktur durch die innere und äußere Membran führt. Der innere Durchmesser dieser Tunnelstruktur kann je nach Lysebedingungen 40 bis 200 nm oder 500 bis 1000 nm betragen. Durch diesen Tunnel wird das cytoplasmatische Material der Zelle freigesetzt und eine leere Zellhülle mit intakter Morphologie zurückgelassen. Die Verwendung von Bakterienghosts als Totimpfstoffe oder Adjuvanzien sowie die Herstellung rekombinanter Bakterienghosts, die in ihrer Membran heterologe Oberflächenproteine tragen, wird in WO 91/13555 und WO 93/01791 beschrieben.

Weiterhin können Ghosts auch aus gram-positiven Bakterien durch Verwendung eines chimären E-L-Lysegens hergestellt werden (US-A-5,075,223).

In DE 199 07 770.4 wird vorgeschlagen, Wirkstoffe in Bakterienghosts zu verpacken. Aufgrund der Löcher in den Ghostmembranen ist eine Retention der Wirkstoffe innerhalb der Ghosts oftmals nur durch aufwändige Maßnahmen erreichbar.

PCT/EP01/00864 beschreibt ein Verfahren zum Verschließen von Bakterienghosts unter Verwendung von Membranvesikeln. Die Fusion der Ghosts mit den Membran-Vesikeln erfolgt durch Inkontaktbringen von kompetenten Ghosts mit Membran-Lipidvesikeln, vorzugsweise in Gegenwart von zweiwertigen Metallkationen oder/und organischen Aggregationsmitteln.

Die der vorliegenden Erfindung zugrunde liegende Aufgabe bestand darin, ein Verfahren zum Verschließen von Bakterienghosts bereitzustellen, welches mit einer höheren Effizienz als bekannte Verfahren funktioniert. Diese Aufgabe wird durch ein Verfahren gelöst, bei dem die Herstellung von verschlossenen Bakterienghosts, vermittelt durch spezifische Wechselwirkungen zwischen Partner eines Bindepaares, erfolgt.

Ein Gegenstand der vorliegenden Erfindung ist somit ein Verfahren zum Herstellen von verschlossenen Bakterienghosts, umfassend das Inkontaktbringen von Bakterienghosts mit Trägermaterialien unter Bedingungen, bei denen ein Verschluss der Bakterienghosts erfolgt, dadurch gekennzeichnet, dass der Verschluss über spezifische Wechselwirkungen zwischen den Partnern eines bioaffinen Bindepaares vermittelt wird, die auf den Ghosts oder/und den Trägermaterialien verankert sind.

Überraschenderweise wurde festgestellt, dass sich die Effizienz des Verschließens von Bakterienghosts durch Trägermaterialien, wie etwa Lipidvesikel, erheblich verbessern lässt, wenn dieses über spezifische Wechselwirkungen zwischen den Partnern eines bioaffinen Bindepaares vermittelt wird. Bevorzugte Beispiele für Partner von bioaffinen Bindepaaren sind Biotin bzw. Biotinanaloga/Avidin bzw. Streptavidin; Haptene/ Antikörper bzw. Antikörperfragmente; Saccharide/Lectine und allgemein Liganden/Rezeptoren. Besonders bevorzugt als Partner von bioaffinen Bindepaaren sind Biotin/Streptavidin. Vorzugsweise wird ein bioaffines Bindepaar verwendet, bei welchem die Partner eine Bindekonstante von mindestens 10⁻⁶ I/mol aufweisen.

Um einen Verschluss zu gewährleisten, liegt zweckmäßigerweise zumindest ein Partner des bioaffinen Bindepaares immobilisiert auf der Membran der Bakterienghosts oder/und auf dem Trägermaterial, z.B. auf der Membran der Lipidvesikel, vor. In einer ersten bevorzugten Ausführungsform ist der erste Partner des bioaffinen Bindepaares (P1, z.B. Biotin) auf der Membran der Bakterienghosts immobilisiert und der zweite Partner des bioaffinen Bindepaares (P2, z.B. Avidin oder Streptavidin) ist auf dem Trägermaterial immobilisiert, so dass der Verschluss der Bakterienghosts einer Wechselwirkung P1-P2 folgen kann. Alternativ kann auch Streptavidin auf der Ghostmembran und Biotin auf dem Trägermaterial immobilisiert sein.

In einer weiteren bevorzugten Ausführungsform ist der erste Partner des bioaffinen Bindepaares (P1) sowohl auf der Membran der Bakterienghosts als auch auf dem Trägermaterial immobilisiert und der zweite Partner des bioaffinen Bindepaares (P2) liegt in freier Form vor, so dass der Verschluss der Bakterienghosts über eine Wechselwirkung P1-P2-P1 erfolgen kann.

Besonders bevorzugt ist ein Partner des bioaffinen Bindepaares mit dem in der Ghostmembran lokalisierten E-Lyseprotein fusioniert, z.B. als C-terminale Fusion. Beispiele bevorzugter Fusionspartner sind Peptide, wie etwa *in vivo* von einem Enzym, z.B. BirA, erkannte Biotinylierungssequenzen, Strep-tag-Sequenzen (Strepatavidin-bindende Peptidsequenzen), Peptidepitop-Sequenzen, d.h. durch einen Antikörper erkannte Peptidepitope, z.B. das FLAG-Epitop, für Metallionen spezifische Peptidsequenzen, z.B. eine für Ni-Ionen spezifische poly-His-Sequenz, oder Polypeptide, wie etwa Streptavidin oder Avidin. Es ist jedoch auch möglich, einen Partner des bioaffinen Bindepaares auf andere Weise in der Membran zu verankern, z.B. durch Fusionen mit anderen Oberflächenproteinen in der Ghostmembran, z.B. den Lyseproteinen E aus dem Phagen ΦX174 und L aus dem Phagen MS2 oder Deletionsvarianten davon, z.B. E' und L'.

Die Verankerung eines Partners von bioaffinen Bindepaaren auf dem Trägermaterial kann - im Falle einer Vesikelmembran - ebenfalls durch Fusionen mit Oberflächenproteinen oder ansonsten durch kovalente Kopplung mittels geeigneter Reagenzien erfolgen.

Im Folgenden sind bevorzugte Ausführungsformen für das erfindungsgemäße Verfahren am Beispiel des bioaffinen Bindepaares Streptavidin/Biotin dargestellt:
(i) biotinylierte Ghosts (z.B. Einführung von Biotingruppen durch eine *in vivo* Biotinylierungssequenz (ivb), z.B. als Eivb-Fusion) + Streptavidin + biotinylierte Vesikel (Einführung einer *in vivo* Biotinylierungssequenz, z.B. als E'ivb- oder L'ivb-Fusion),
(ii) biotinylierte Ghosts + Streptavidin-Vesikel (z.B. E'-Streptavidin-Fusion),
(iii) Streptavidin-Ghosts (z.B. E'-Streptavidin-Fusion) + biotinylierte Vesikel (z.B. E'ivb-Fusion oder L'ivb-Fusion)
(iv) biotinylierte Ghosts + Streptavidin + biotinylierter S-Layer (z.B. SbsA-ivb-Fusion),
(v) Streptavidin-Ghosts (z.B. E'-Streptavidin-Fusion) + biotinylierter S-Layer (z.B. SbsA-ivb-Fusion),
(vi) biotinylierte Ghosts (E-ivb-Fusion) + Streptavidin + biotinylierte Liposomen,
(vii) Streptavidin-Ghosts (z.B. E'-Streptavidin-Fusion) + biotinylierte Liposomen.

Das erfindungsgemäße Verfahren kann eine zumindest teilweise Fusion der Membran der Bakterienghosts und der Membran von Lipidvesikeln umfassen, die bevorzugt als Trägermaterial verwendet werden. In anderen Ausführungsformen ist eine Membranfusion jedoch nicht erforderlich. Bevorzugte Bedingungen für eine Membranfusion sind in PCT/EP 01/00864 offenbart und umfassen die Bereitstellung kompetenter Bakterienghosts, z.B. durch Inkontaktbringen von Ghosts mit zweiwertigen Metallkationen, insbesondere Calciumionen, und anschließender Inkubation bei einer tiefen Temperatur, z.B. von 0-5 °C. Die Fusion erfolgt vorzugsweise unter Bedingungen, bei denen sowohl die Membran der Bakterienghosts als auch die Membran der Lipidvesikel in einem flüssigen Zustand ist, z.B. bei einer Temperatur von ≥30 °C. Um eine effiziente Fusion zu erzielen, werden die Membranen in engen Kontakt gebracht, so dass elektrostatische Abstoßungskräfte zwischen den Bakterienghosts und den Lipidvesikeln überwunden und die Membranen in den Ausgangsmaterialien destabilisiert werden, z.B. während einer Ultrazentrifugation oder durch Verwendung chemischer Fusogene, wie Polyethylenglykol, Glycerin, DMSO oder/und Polyhistidin. Besonders bevorzugt erfolgt die Fusion zwischen den Membranen der Bakterienghosts und der Lipidvesikel in Gegenwart von Hilfsmitteln, z.B. zweiwertigen Metallkationen, insbesondere Calciumionen, oder/und organischen Aggregationshilfsmitteln.

Besonders bevorzugt stammen die Ghosts von gram-negativen Bakterien, die z.B. ausgewählt werden aus Escherichia coli, z.B. enterohämorrhagischen (EHEC) und enterotoxigenen E. coli Stämmen, Klebsiella, Salmonella, Enterobacter, Pseudomonas, Vibrio, Actinobacillus, Haemophilus, Pasteurella, z.B. P. haemolytica oder P. multocida, Bordetella, z.B. B. bronchiseptica oder B. pertussis, Helicobacter, Francisella, Brambamella, Erwinia, Ralstonia, Pantoea, Streptomyces, Frankia, Serratia, Agrobacterium, Azotobacter, Bradyrhizobium, Burkholderia, Rhizobium, Rhizomonas und Sphingomonas. Besonders bevorzugte Beispiele für grampositive Bakterien sind Staphylococcus, Streptococcus und Bacillus.

Es können auch Ghosts eingesetzt werden, die von rekombinanten Bakterien stammen und heterologe Membranproteine enthalten. Diese Ghosts mit modifizierten Hüllen sind insbesondere für eine human- oder veterinärmedizinische Verabreichung bedeutsam, die ein Targeting, d.h. einen Transport der Ghosts an Zielzellen oder Zielgewebe, erfordert. Hierzu können modifizierte Ghosts eingesetzt werden, die targetspezifische Oberflächenmoleküle an ihrer Membranaußenseite tragen. Die Einführung dieser targetspezifischen Oberflächenmarker, wie etwa Zucker, z.B. Mannose oder Fucose, oder Proteine, wie Invasin aus Yersinien oder Invasinderivaten, kann durch rekombinante Expression entsprechender membranständiger Fusionspolypeptide in der Bakterienzelle vor der Lyse oder/und durch Anlagerung an die Membran mittels eines geeigneten Rezeptorsystems, z.B. Streptavidin/Biotin, erfolgen.

Die Trägermaterialien können aus natürlichen oder synthetischen Quellen stammen und sind vorzugsweise Lipidvesikel, besonders bevorzugt Lipidvesikel mit einer Lipid-Doppelschicht, die Phospholipide, wie etwa Phosphatidylethanolamin, enthält. Beispielsweise können Vesikel eingesetzt werden, die bei Homogenisierung von Zellen, insbesondere Bakterienzellen, z.B. durch Ultraschall, oder in einer Frenchpress entstehen. In diesem Zusammenhang können sogenannte Inside-Out-Vesikel (Innenmembran nach außen gekehrt) oder Rightside-Out-Vesikel (Membranorientierung bleibt erhalten) eingesetzt werden, wobei Inside-Out-Vesikel bevorzugt sind. Inside-Out-Vesikel können nach Homogenisierung von Zellen durch bekannte Methoden, z.B. Gradientenzentrifugation, angereichert werden.

Andererseits können auch synthetische Lipidvesikel, wie Liposomen, eingesetzt werden. Weiterhin als Lipidvesikel geeignet sind membranumhüllte Viren wie etwa Pox-, Chordopox-, Herpes-, Hepadnaviridae (DNA-Viren) sowie Corona-, Paramyxo-, Bunya-, Orthomxyxo-, Arena-, Toga-, Flavi-, Retro-, Rhabdoviridae (RNA-Viren). Selbstverständlich können auch Kombinationen der genannten Lipidvesikel eingesetzt werden. Andere geeignete Beispiele für Trägermaterialien sind Polymerpartikel, z.B. organische Polymer- oder Copolymerpartikel, anorganische Partikel, Biopolymerpartikel, z.B. Saccharide oder Verbundmaterialien. Die Größe der Trägerpartikel liegt vorzugsweise im Bereich eines mittleren Durchmessers von 20-2000 nm und kann entsprechend den verwendeten Trägermaterialien und der Größe der Löcher in der Ghostmembran variiert werden.

Ein wichtiger Aspekt der Erfindung beinhaltet das Verpacken von Wirkstoffen in den geschlossenen Bakterienghosts. Der Wirkstoff kann jeder beliebige, in das Innere der Bakterienghosts transportierbare und dort gegebenenfalls immobilisierbare Wirkstoff sein. Beispiele für Wirkstoffe sind pharmakologisch wirksame Substanzen, Markierungssubstanzen, landwirtschaftlich wirksame Substanzen, z.B. Vakzine, Farbstoffe, aber auch genetisches Material und Zellkomponenten, z.B. Zellextrakte, Bestandteile von Zellextrakten oder Zellorganellen wie Ribosomen. Gegebenenfalls können mehrere Wirkstoffe gemeinsam verpackt werden, z.B. zur Herstellung von Kombinationsvakzinen.

Die Verpackung der Wirkstoffe kann auf unterschiedliche Art und Weise erfolgen. So können die Wirkstoffe vor dem Verpacken in die Ghosts eingebracht werden und dort gegebenenfalls immobilisiert werden. Weiterhin können die Wirkstoffe auch in gelöster Form im Verpackungsmedium vorhanden sein. Darüber hinaus ist es möglich, die Wirkstoffe in die zur Fusion mit den Ghosts verwendeten Lipidvesikel zu verpacken. Verfahren zum Verpacken von Wirkstoffen in Lipidvesikel sind bekannt, siehe z.B. J. Treat et al., Liposomes in the Therapy of Infections, Diseases and Cancer, G. Lopez-Berestein und I.J. Fidler, Eds. (Liss, New York, 1989), S. 353 - 365, (Doxorubicin); G. Lopez-Berestein ibid., S. 317 - 327 (Amphotericin B); E.S. Kleineman et al., Cancer Res. 49: 4665 (1989), G. Poste et al., ibid. 42, 1412 (1982); G.R. Alving et al., Vaccine 4, 166 (1986) (Vakzine); A.G. Allison und G. Gregoriadis, Nature 252: 252 (1974) (Vakzine); V.V. Ranade, J. Clin. Pharmacol. (1989) 29: 685 - 694; S.S. Davis, Drugs Exp. Clin. Res. (1985) 11: 633-640; T.M. Allen, Drugs (1998) 56: 747 - 756; P.P. Speiser, Methods Find Exp. Clin. Pharmacol. (1991) 13: 337 - 342; R. Singh und S.P. Vyas, J. Dermatol. Sci. (1996) 13: 107 - 111; P.N. Shek et al., J. Drug Target (1994) 2: 431 - 442; Z. Pavelic et al., Eur. J. Pharm. Sci. (1999) 8: 345- 351; J. M. Sollovitz et al., Vet. Res. (1998) 29: 409 - 430 sowie die darin zitierten Literaturstellen.

Beispiele für pharmakologisch wirksame Substanzen sind Polypeptide wie etwa Antikörper, therapeutisch wirksame Polypeptide wie Zytokine, Interferone, Chemokine etc., Enzyme und immunogene Polypeptide oder Peptide. Ein weiteres Beispiel für Wirkstoffe sind Nukleinsäuren, z.B. DNA oder/und RNA, insbesondere therapeutische Nukleinsäuren, z.B. Nukleinsäuren für die Gentherapie, die vorzugsweise in Form eines chromosomal integrierbaren Vektors vorliegen, oder Nukleinsäuren für eine Nukleinsäure-Vakzinierung; Antisense-Nukleinsäuren oder Ribozyme. Noch weitere Beispiele für Wirkstoffe sind niedermolekulare Wirksubstanzen, Peptide, Hormone, Antibiotika, Antitumormittel, Steroide, Immunmodulatoren etc. Die Wirkstoffe können in den Bakterienghosts in gelöster Form, als Suspensionen oder/und als Emulsionen gegebenenfalls in Kombination mit geeigneten Träger- oder/und Hilfsstoffen vorliegen. Weiterhin können die Wirkstoffe auch diagnostische Markierungssubstanzen, z.B. fluoreszierende Substanzen, Farbstoffe oder Röntgenkontrastmittel sein.

Auch nichtmedizinische Wirkstoffe können in Ghosts verpackt werden, z.B. Wirkstoffe aus dem Agrarbereich wie Insektizide, Herbizide, Mittel gegen Nematoden, Enzyme zur Bodenverbesserung, Dünger, Wachstumsförderer, wasserbindende Proteine zur besseren Durchfeuchtung oder Wasserbindung unter atmosphärischen Bedingungen. Andere Anwendungen sind die Verpackung von Farbstoffen für die Druckindustrie, z.B. fälschungssichere Tinten mit immunologischer Nachweismöglichkeit, und die Verpackung von Vitaminen oder Probiotika für die Lebensmittelindustrie. Ebenso möglich ist die Verpackung von kosmetischen Mitteln oder von Stoffen wie Salzen oder anderen ionischen Substanzen.

Der Wirkstoff kann in den Bakterienghosts in immobilisierter Form vorliegen. Die Immobilisierung des Wirkstoffs kann mittels kovalenter oder nichtkovalenter Wechselwirkungen, z.B. elektrostatischer Wechselwirkungen, hochaffiner biologischer Wechselwirkungen, durch mechanische Retention oder eine Kombination von zwei oder mehreren der genannten Möglichkeiten erfolgen.

Alternativ kann der Wirkstoff innerhalb des Ghosts auch in freier Form vorliegen, da nach dem Verschließen der Ghosts ein Verlust des Wirkstoffs durch die Membran hindurch im wesentlichen ausgeschlossen ist.

Auch Kombinationen von immobilisierten Wirkstoffen und Wirkstoffen in freier Form können durch das erfindungsgemäße Verfahren verpackt werden.

Ein weiterer Gegenstand ist somit ein verschlossener Bakterienghost, der durch das zuvor beschriebene Verfahren erhältlich ist. Der verschlossene Bakterienghost kann eine zunächst teilweise intakte Membran enthalten, d.h. eine das Innere des Ghosts von der Umgebung trennende kontinuierliche Lipidschicht, vorzugsweise eine kontinuierliche Lipid-Doppelschicht. Die verschlossenen Bakterienghosts können verkapselte Wirkstoffe enthalten, metabolische Funktionen aufweisen oder/und die Fähigkeit zur Proliferation besitzen. Die verschlossenen Bakterienghosts können gegebenenfalls auch gefriergetrocknet weredn, um die Haltbarkeit zu verbessern.

Je nach Abstand zwischen dem Bakterienghost und dem Trägermaterial kann die Dichte des Verschlusses und somit die Freisetzungsrate der verpackten Substanzen moduliert werden. So können beispielsweise - um den Abstand zu erhöhen - freie Bindepartner P1 und P2, z.B. Biotin und Streptavidin, zugesetzt werden, um "kaminartige" Strukturen mit vergrößertem Abstand zwischen Ghostmembran und Trägermaterial zu bilden.

Weiterhin soll die Erfindung durch die nachfolgenden Figuren und Beispiele erläutert werden. Es zeigen:
**Figur 1** **Herstellung von Expressionsplasmiden**

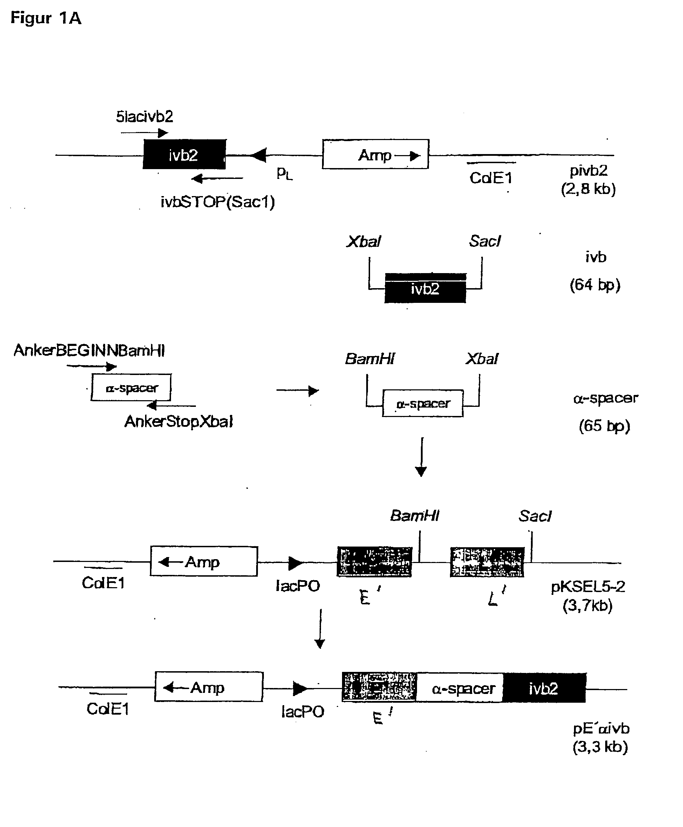

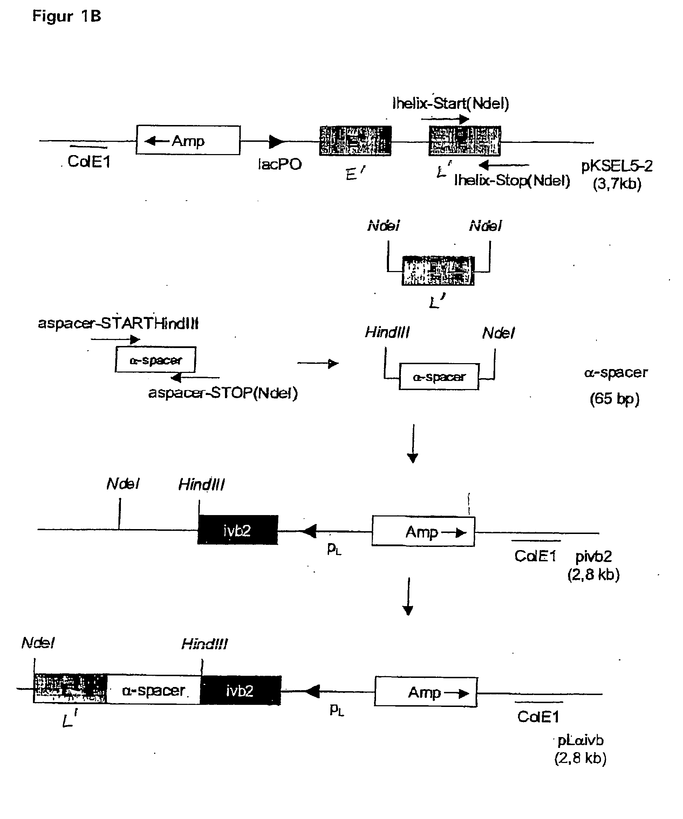

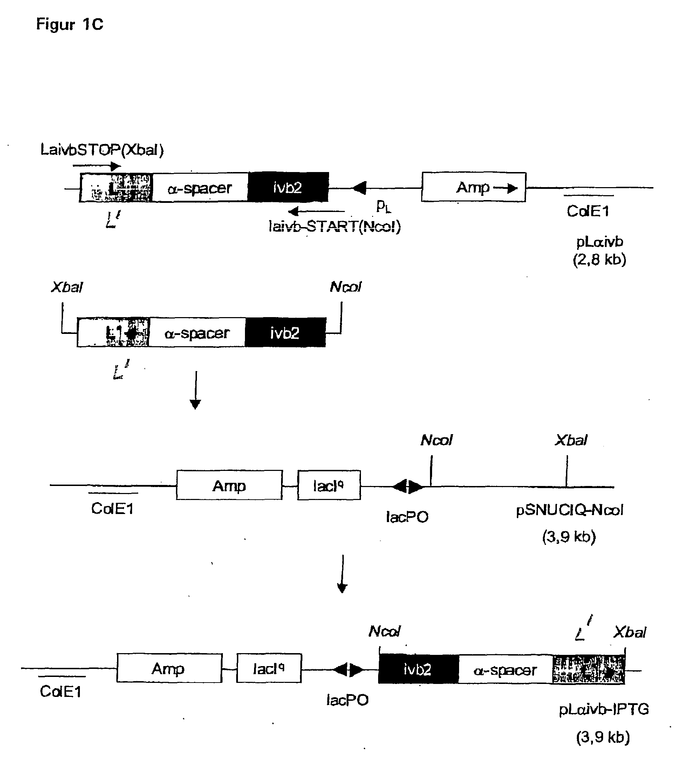

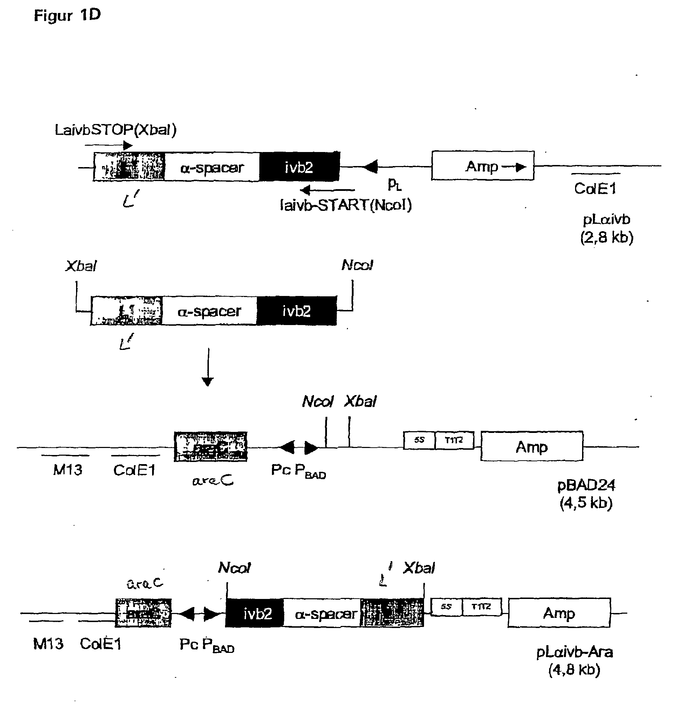

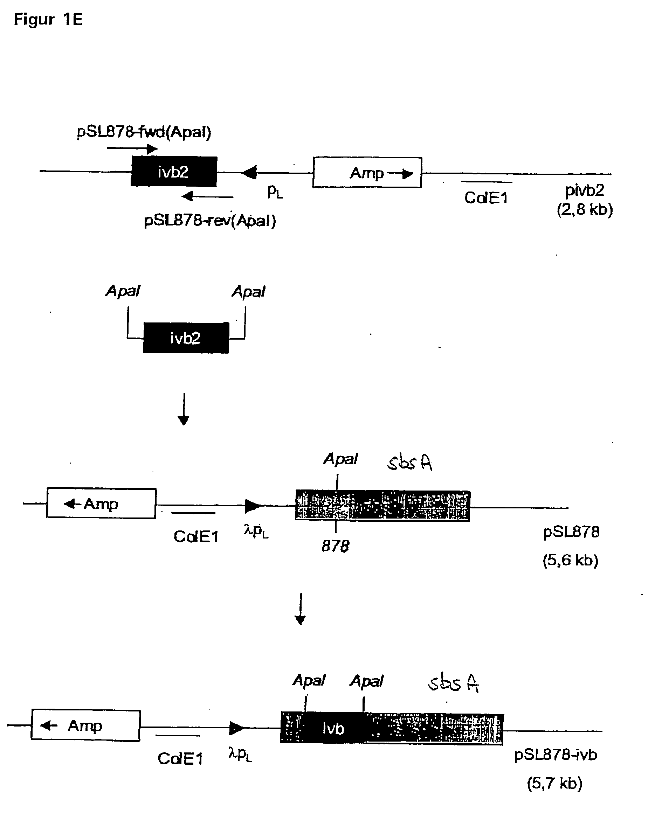

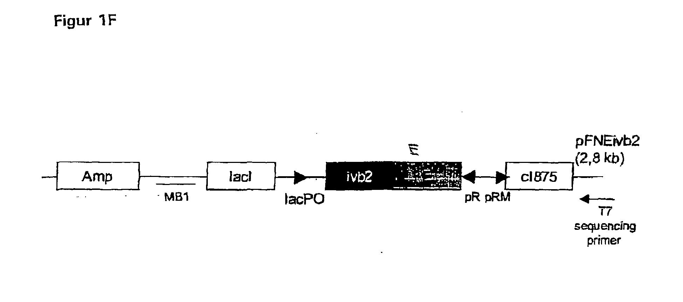

   **Figur 1A** zeigt die Herstellung des Plasmids pE'αivb, welches ein E'αivb-Fusionsprotein exprimiert. E' enthält die 54 N-terminalen Aminosäuren des Lyseproteins E. **Figur 1** **B** zeigt die Herstellung des Plasmids L'αivb, welche ein L'ivb-Fusionsprotein exprimiert. L' enthält die 56 C-terminalen Aminosäuren des Lyseproteins L. **Figur 1C** zeigt die Herstellung des Plasmids pL'αivb-IPG, welches ein L'αivb-Fusionsprotein unter regulierbarer Kontrolle des lac-Promotor/Operatorsystems exprimiert. **Figur 1** **D** zeigt die Herstellung des Plasmids pL'αivb-Ara, das ein L'αivb-Fusionsprotein unter Kontrolle des durch Arabinose regulierbaren Promotors P_{BAD} (Promotor des Ara-Operons von E. coli) exprimiert. **Figur 1E** zeigt die Herstellung des Plasmids pSL878ivb, das ein S-Layer (SbsA) ivb-Fusionsprotein unter Kontrolle des ^pL-Promotors exprimiert, wobei die ivb-Sequenz in die Apal-Restriktionsstelle an Position 878 des SbsA-Gens inseriert ist. **Figur 1** **F** zeigt schließlich das Plasmid pFNEivb2, das ein Eivb-Fusionsprotein unter Kontrolle des lac-Promotors/Operators exprimiert.
**Figur 2** **Gerichtete Fusion durch biotinylierte Ghosts und Streptavidin-Membranvesikel**
   Biotinylierte Ghosts werden durch Expression eines Eivb-Fusionsproteins (ivb = in vivo Biotinylierungssequenz) erhalten. Streptavidin-tragende Membranvesikel werden durch Expression eines E'-StrpA-Fusiongens in Bakterienzellen und anschließender Vesikelpräparation erhalten. Bei E' handelt es sich um die 54 N-terminalen Aminosäuren des E-Proteins, die hier als Membrananker Verwendung finden.

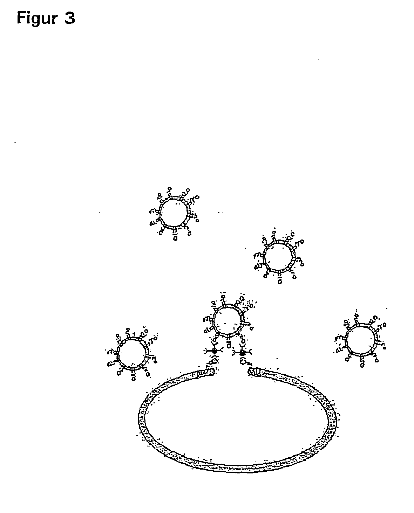

**Figur 3** **Gerichtete Fusion durch biotinylierte Ghosts, freies Streptavidin und Biotin-Membranvesikel**
   Biotinylierte Ghosts werden durch Expression eines E-ivb-Fusionsproteins (ivb = *in vivo* Biotinylierungssequenz) erhalten. Biotinylierte Membranvesikel werden durch Expression des E'-ivb-Fusiongens und anschließender Vesikelpräparation erhalten. Als Brücke zum biotinylierten E-Protein wird Streptavidin eingesetzt.

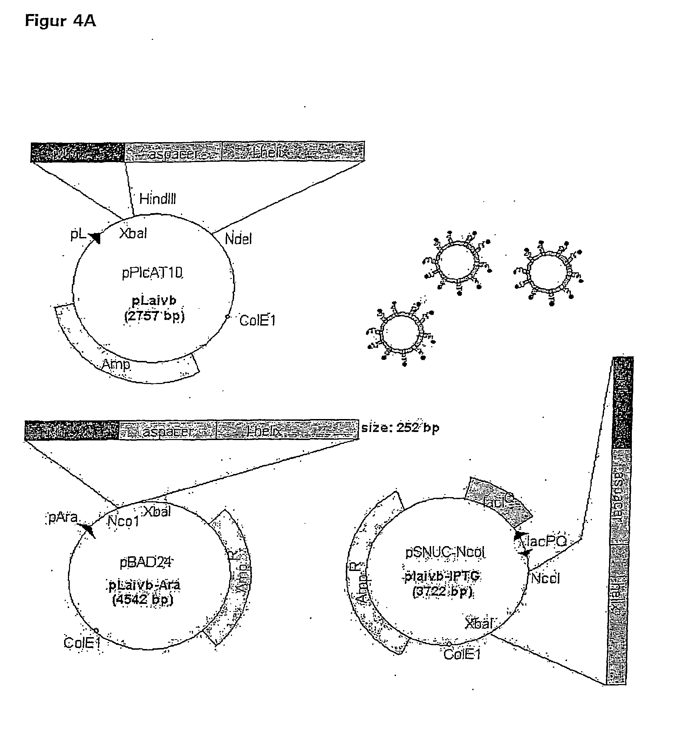

**Figur 4A** **Expressionsplasmide zur Erzeugung von Biotin-tragenden Membranvesikeln**
   Biotinylierte Membranvesikel werden durch Expression eines L'-ivb-Fusiongens und anschließender Vesikelpräparation erhalten. Bei L' handelt es sich um die 56 C-terminalen Aminosäuren des L-Proteins, die hier als Membrananker Verwendung finden. Als Transkriptionsregulationseinheiten werden hier pL (Lambda Promotor), pAra (Arabinose Promotor) und lacPO (Lactose Promotor/Operator) verwendet. Alle Plasmide tragen den ColE1 Replikationsursprung und das Ampicillinresistenzgen. Die 252 bp große Membran-Biotinylierungssequenz weist zwischen Membrananker (L-Helix) und Biotinylierungssequenz (ivb) einen 45 bp Abstandshalter (α-spacer) auf.

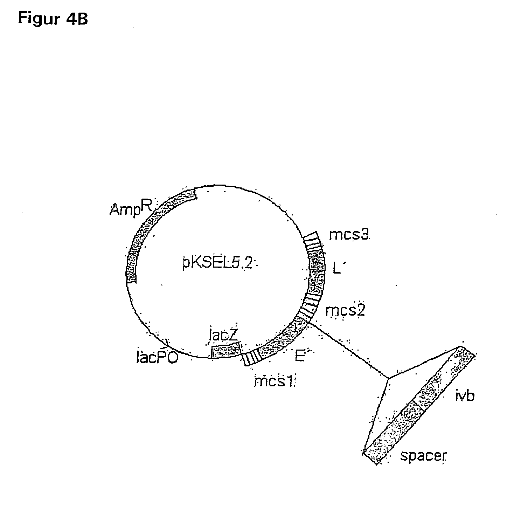

**Figur 4B** **Expressionsplasmid zur Erzeugung von Biotin-tragenden Membranvesikeln**
   Biotinylierte Membranvesikel werden durch Expression eines E'-ivb-Fusiongens und anschließender Vesikelpräparation erhalten. Bei E' handelt es sich um die 54 N-terminalen Aminosäuren des E-Proteins, die hier als Membrananker Verwendung finden. Als Transkriptionsregulationseinheiten wurde hier der lacPO (Lactose Promotor/Operator) verwendet.

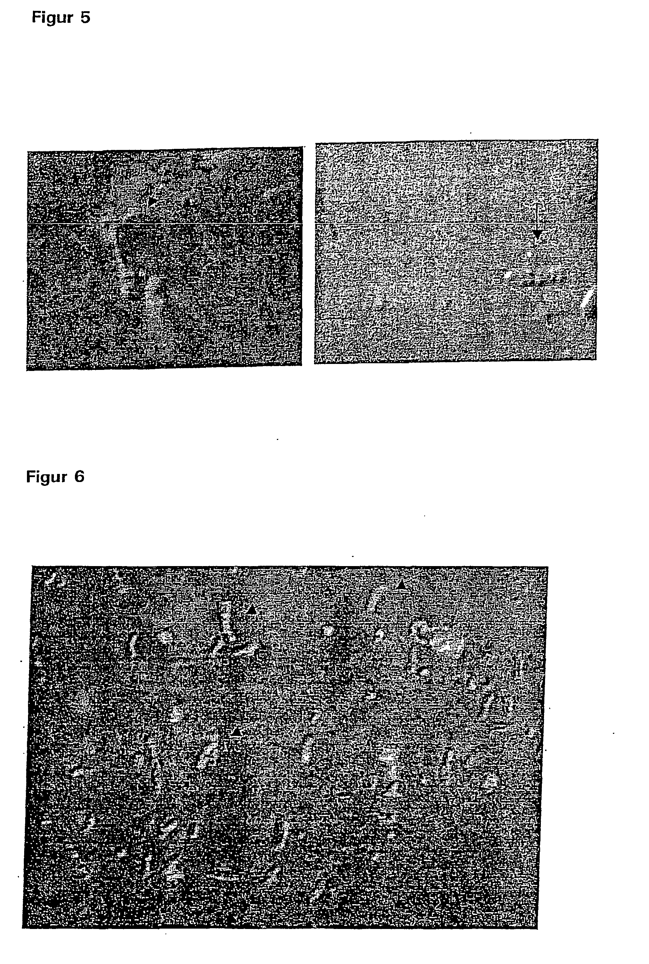

**Figur 5** **Mikroskopische Aufnahmen des Verschlusses von E. coli-Ghosts mit Membranvesikeln über bioaffine Wechselwirkungen**
   Die Pfeile kennzeichnen die Lage eines Membranvesikels an der Polkappe oder der Zellteilungsebene.
**Figur 6** **Mikroskopische Aufnahmen des Verschlusses von E. coli-Ghosts mit calceinbeladenen Membranvesikeln über bioaffine Wechselwirkungen**
   Die fluoreszierenden, weil calceingefüllten Vesikel, tragen biotinylierte Membrananker und werden durch zugegebenes Streptavidin an die biotinylierten Lyselöcher der Ghosts dirigiert. Dort kommt es zu einer Fusion, wobei das Calcein teilweise auch in die Ghosts einfließt und diese in ihrer Gesamtheit fluoreszieren lässt (Pfeile).

### 1. Material und Methoden

### 1.1. Herstellung von Expressionsvektoren

Die kodierenden Sequenzen für ein Minimalpeptidsubstrat zur *in vivo-*Biotinylierung (ivb) (Beckett et al., Protein Sci 8 (1999), 921-929; Schatz, Biotechnol. 11 (1993), 1138-1143) und für ein α-helikales Peptid (α-Spacer; Aminosäuresequenz GGAAAAKAAAAKAAAAKGG (SEQ ID NO. 1); Chakrabarty et al., Biochemistry 32 (1993), 5560-5565; Vila et al., PNAS USA 89 (1992), 7821-7825 und Padmanabhan und Baldwin, Protein Sci. 3 (1994), 1992-1997) wurden nach Standardmethoden (Sambrook und Russel, Molecular Cloning, A Laboratory Manual (2001) 3. Ed., Cold Spring Harbor Laboratory Press) in den Vektor pKSEL5-2 (Szostak et al., J. Biotechnol. 44 (1996), 161-170) kloniert, der nach Restriktionsspaltung mit BamHI und SacI für den E'-Anker kodiert (siehe Figur 4B).

Der α-Spacer wurde durch PCR unter Verwendung der komplemetären Primer A1/B1(A1:5'GGTGGTGCAGCAGCAGCAAAAGCGGCCGCGGCCAA A3' (SEQ ID NO. 2); B1:5'ACCACCTTTAGCAGCAGCAGCTTTGGCCGC GGCCCGCTTT3' (SEQ ID NO. 3) amplifiziert. Eine zweite Amplifikation mit 0einem Primer A2 zur Einführung der Restriktionsstelle für BamHI und einem Primer B2 zur Einführung einer Restriktionsstelle für Xbal wurde anschließend durchgeführt (A2:5'CAGC AGGGATCCCGGGTGGTGCAGC AGCAGCAT3' (SEQ ID NO. 4); B2:5'CAGCAGTCTAGAA CCACCTTTAGC AGCAGCAG3' (SEQ ID NO. 5)). Das α-Spacer PCR-Produkt wurde mit BamHI am N-terminalen Ende und mit Xbal am C-terminalen Ende geschnitten. Die Biotinylierungssequenz wurde aus dem Plasmid pivb (Mayrhofer (2003), Immobilization of DNA in bacterial ghosts, Doktorarbeit, Universität Wien, Österreich) unter Verwendung des Primers P1 zur Einführung einer Xbal-Restriktionsstelle am 5'-Ende und des Primers P2 zur Einführung - einer Sacl-Restriktionsstelle am 3'-Ende amplifiziert (P1:5'CAGCAGTCTAGAGGTGG TGGTCTGAACGACATCTTCG3' (SEQ ID NO. 6)) (P2:5'CAGCAGGAGCTCGGTGGTGGTCTGAACGACATCTTCG3' (SEQ ID NO. 7)). Das resultierende Plasmid PE'αivb kodiert für ein Fusionsprotein, das am N-Terminus die E'-Sequenz und daran anschließend den α-Spacer und die ivb-Sequenz enthält.

Auf analoge Weise wurde das Expressionsplasmid pL'αivb hergestellt, das ein Fusionsprotein aus dem L'-Membrananker des Phagen MS2 (56 C-terminale Codons des Lyseproteins L) fusioniert an den α-Spacer und die ivb-Sequenz exprimiert, wobei die ivb-Sequenz am N-Terminus des Fusionsproteins angeordnet ist (Figur 1 B). Aus pLα'ivb wurden weiterhin die Plasmide pL'αivb-IPTG und pL'αivb-Ara hergestellt, die das L'αivb-Fusionsprotein unter Kontrolle von durch IPTG bzw. Arabinose regulierbaren Promotoren exprimieren (Figuren 1C und 1D).

Aus dem Plasmid pSL878 (Hovorka et al., FEMS Microbiol. Lett. 172 (1999), 187-196; Kuen et al., Mol. Microbiol. 19 (1995), 495-503) wurde durch Insertion der *in vivo* Biotinylierungssequenz ivb an Position 878 des S-Layer sbsA Gens das Plasmid pSL878ivb hergestellt (Figur 1 E).

Das in Figur 1F gezeigte Plasmid pFNEivb2 wurde, wie in Mayrhofer (2003, supra) beschrieben, hergestellt.

### 1.2 Expression von E'αivb und L'αivb und Herstellung von Membranvesikeln

E.coli NM522-Zellen, transformiert mit den Plasmiden pE'αivb oder pL'αivb-IPTG wurden in 2 I LB-Medium unter Zusatz von Ampicillin (100 µg/ml) und 1 mM IPTG (Isopropyl β-D-thioglucopyranosid) bei 37 °C für 6 h kultiviert. Die Zellen wurden geerntet, mit PBS, pH 7,4 gewaschen in 40 ml PBS aufgenommen und bei -70 °C aufbewahrt.

Ein Western-Blot unter Verwendung eines Streptavidin-Meerettich-Peroxidase-Konjugats und des SuperSignalR West Chemilumineszent Substrate (Pierce) zur Entwicklung bestätigten die Expression von E'αivb und L'αivb. Nach der Methode von de Leij und Witjold (Biochem. Biophys. Acta 471 (1977), 92-104) wurden Membranvesikel hergestellt. Resuspendierte Bakterien wurden durch eine French-Presse (SL Aminco, USA) bei 14400 psi einmal geleitet. Zellfragmente und Zellrückstände wurden durch zweimalige Zentrifugation bei 12000 U/min für 10 min entfernt.

Im Überstand vorhandene Vesikel wurden durch Zentrifugation bei 285000 g für 1 h pelletiert und in 2 ml Tris-Puffer (10 mM, pH 7,5) resuspendiert. Diese Membranvesikelpräparation wurde mit Saccharose (Endkonzentration 22 % v/v) versetzt und auf einen diskontinuierlichen Saccharosegradienten (60 % - 25 % in 5 %-Schritten) geschichtet. Eine Zentrifugation des Gradienten bei 4 °C für 16 h (40000 u/min, SW40Ti-Rotor) führte zu einer Auftrennung der Inside-Out und Rightside-Out-Vesikel, die nach einmaligem Waschen in PBS aufgenommen wurden.

Alternativ wurden Inside-Out und Rightside-Out-Vesikel in PBS versetzt mit Calcein (2',7'-Bis[N,N-bis(carboxymethanol)-aminomethyl]fluorescin) aufgenommen, durch eine Nylonmembran (Porengröße 200 nm) eines Extruders (LiposoFast-Basic, Avestin, Kanada) geleitet und einmal gewaschen, um nicht eingeschlossenes Calcein zu entfernen. Schließlich wurden die Vesikel in PBS aufgenommen.

### 1.3 Herstellung von Ghosts, die in vivo biotinyliertes Lyseprotein E exprimieren

Ghosts wurden aus E.coli NM 522 transformiert mit dem Plasmid pFNEivb2K/7 (Figur 1 F) hergestellt. Hierzu wurden die Bakterien in LB-Medium versetzt mit Ampicillin bis zu einer optischen Dichte von 0,3 bei 28 °C kultiviert. Dann erfolgte ein Temperaturshift auf 42 °C, um eine Expression des Lyseproteins E und eine Lyse zu induzieren. Die Lyse wurde bis zum Erreichen einer OD600 von 0,07 durchgeführt. Dann wurden die Ghosts geerntet und, mit PBS, pH 7,4 gewaschen, in PBS aufgenommen und gefroren bei -20 °C aufbewahrt. Die Expression und *in vivo* Biotinylierung wurde durch Western Blot-Analyse unter Verwendung eines Streptavidin-Meerrettich-Peroxidase-Konjugats überprüft.

### 1.4 Herstellung von in vivo biotinyliertem S-Layer Protein

*In vivo* biotinyliertes S-Lyer Protein SbsA wurde nach Expression des Plasmids pSL878ivb in E. coli nach Induzierung der Expression durch Temperaturänderung von 28 °C auf 42 °C nachgewiesen. Es konnte durch Dot-Blot-Analyse gezeigt werden, dass das *in vivo* biotinylierte SbsAivb-Fusionsprotein mit Streptavidin reagieren kann.

### 1.5 Verschließen von Ghosts mit einem Membranvesikeln über eine Biotin-Streptavidin-Wechselwirkung

Biotinylierte Ghosts wurden mit einem Überschuss von Streptavidin (>1,38 µg Streptavidin pro mg Ghost-Protein) inkubiert. Nach 30 min Inkubation wurde nicht gebundenes Streptavidin durch Zentrifugation und Waschen mit PBS entfernt. Inside-Out und Rightside-Out Vesikel wurden zugegeben und 30 min bei Raumtemperatur inkubiert. Nach Entfernen überschüssiger Vesikel durch Zentrifugation bei 12000 U/min (SS34 Rotor) wurden die Ghosts erneut in PBS aufgenommen und mikroskopisch untersucht. Die Fotos wurden bei einer Vergrößerung von 1575 mit Epifluoreszenzmikroskop (Axioplan, Zeiss) und einem Schwarz-Weiß-Fotomultiplier und der entsprechenden Software (Metaview) erstellt.

### 2. Ergebnisse

### 2.1 Verschließen von Ghosts mit Streptavidin-Membranvesikeln

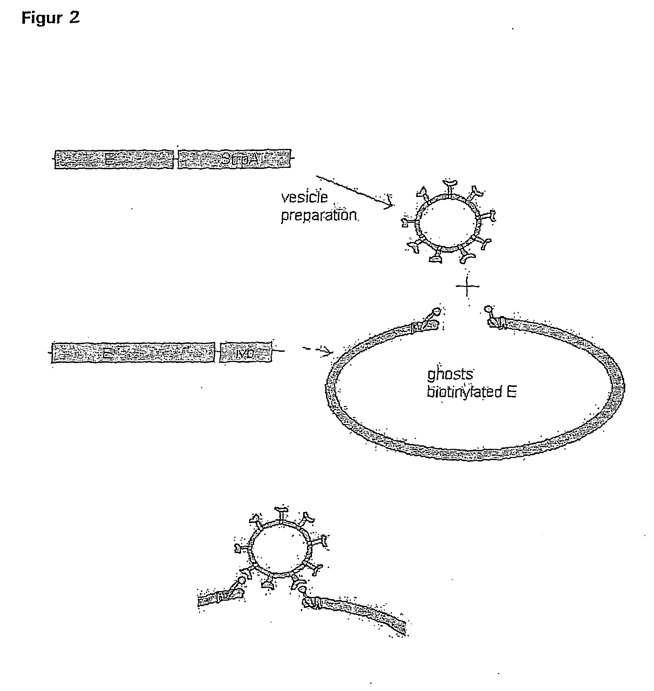

Um eine bessere Verschlusseffizienz von Bakterienghosts zu erreichen, wurden sowohl das Lyseprotein als auch die Membranvesikel so verändert, dass über eine spezifische Rezeptorbindung ein Targeting der Vesikel zum Lysetunnel möglich wird. Dazu wurde eine DNA-Sequenz, die für ein *in vivo-*Biotinylierungssignal (ivb) kodiert, 3'-seitig an das E-Lysegen fusioniert. Das E-Protein wird dadurch bereits in der Zelle biotinyliert, was allerdings die Lyseeigenschaften nicht beeinträchtigt. Solchermaßen hergestellte Ghosts weisen einen Lysetunnel auf, der mit vielen Biotinmolekülen markiert ist, und daher für Membranvesikel, die membranverankerte Streptavidinmoleküle aufweisen, ein bevorzugter Bindepartner ist (Figur 2). Es konnte eine effiziente Verpackung von Calcein in Ghosts gezeigt werden.

### 2.2 Verschließen von Ghosts mit biotinylierten Membranvesikel und eine Streptavidin-Brücke

Es wurden Expressionsvektoren konstruiert, um Vesikel herstellen zu können, die ebenso wie das Lyseprotein biotinyliert sind, und, nach Zugabe von Streptavidin als verbindendes Agens, über Biotin-Streptavidin-Biotin-Wechselwirkung spezifisch an den Lysetunnel andocken können (Figur 3).

Es wurden Expressionsplasmide für die ivb-Sequenz mit Membranankern (L'-ivb, Figur 4A; E'-ivb, Figur 4B) hergestellt, die auch eine unterschiedliche Expressionskontrolle zuließen. Als induzierbare Promotoren wurden der Arabinose-, der Lactose- und der linksseitige Lambda pL-Promotor gewählt. Zusätzlich wurde zwischen der *in vitro*-Biotinylierungssequenz und dem Membrananker ein ein kurzer Abstandhalter eingefügt (α-spacer), um eine bessere Zugänglichkeit des Biotinmoleküls zu gewährleisten.

Die Expressionsprodukte und durch French press erzeugte "Inside-out" und "Rightside-out"-Vesikel wurden im Western Blot untersucht. In E.coli wurden das E'-ivb Protein und das IPTG- oder Arabinose-induzierbare L'-ivb Protein exprimiert. Es kommt zu einer Anreicherung in der inneren Membran (Fraktion der Inside-out Vesikel). Cytoplasmatische Komponenten (darunter das E.coli-eigene Biotincarboxylcarrierprotein BCCP) gehen durch den Pressvorgang verloren. Weiterhin konnte die Zugänglichkeit des Biotins des Lyseproteins wie auch des das Fusionsprotein tragenden Vesikels im Dot blot gezeigt werden.

Im Weiteren wurden E'-ivb-tragende Membranvesikel in Verschlussexperimenten eingesetzt. Auf mikroskopischen Aufnahmen ist sehr gut die Positionierung der modifizierten Membranvesikel über den Ghosts zu erkennen (Figur 5). In allen Fällen waren die Vesikel an Regionen der Ghosts beobachtet worden, wo auch die E-Lysetummel zu beobachten sind. In Kontrollansätzen mit nicht-modifizierten Membranvesikeln waren solche Vesikel-Anlagerungen nicht zu finden.

Bei Inkubation von Ghosts mit modifizierten Membranvesikeln, die zusätzlich mit Calcein beladen waren, konnte auch eine Verlagerung der Fluoreszenz vom Vesikel zum Ghost beobachtet werden (Figur 6). Dies kann durch Fusion des Vesikels mit dem Membransystem der Ghosthülle und einem damit verbundenen Einströmen des Calceins in den Ghosts erklärt werden.

## Patentansprüche

1. Verfahren zum Herstellen von verschlossenen Bakterienghosts, umfassend das Inkontaktbringen von Bakterienghosts mit Trägermaterialien unter Bedingungen, bei denen ein Verschluss der Bakterienghosts erfolgt,
**dadurch gekennzeichnet,**
**dass** die Fusion über spezifische Wechselwirkungen zwischen den Partnern eines bioaffinen Bindepaares vermittelt wird, die auf den Ghosts oder/und den Trägermaterialien verankert sind.

2. Verfahren nach Anspruch 1,
**dadurch gekennzeichnet,**
**dass** die Partner des bioaffinen Bindepaares ausgewählt werden aus der Gruppe bestehend aus Biotin bzw. Biotinanaloga/Streptavidin bzw. Avidin, Hapten/Antikörper bzw. Antikörperfragmente, Saccharid/Lectin und Ligand/Rezeptor.

3. Verfahren nach Anspruch 2,
**dadurch gekennzeichnet,**
**dass** man als bioaffines Bindepaar Biotin/Streptavidin verwendet.

4. Verfahren nach einem der Ansprüche 1 bis 3,
**dadurch gekennzeichnet,**
**dass** zumindest ein Partner des bioaffinen Bindepaares immobilisiert auf der Membran der Bakterienghosts und auf dem Trägermaterial vorliegt.

5. Verfahren nach Anspruch 4,
**dadurch gekennzeichnet,**
**dass** ein erster Partner des bioaffinen Bindepaares (P1) auf der Membran der Bakterienghosts und ein zweiter Partner des bioaffinen Bindepaares (P2) auf dem Trägermaterial immobilisiert ist, und der Verschluss über eine Wechselwirkung P1-P2 erfolgt.

6. Verfahren nach Anspruch 4,
**dadurch gekennzeichnet,**
**dass** ein erster Partner des bioaffinen Bindepaares (P1) auf der Membran der Bakterienghosts und dem Trägermaterial immobilisiert ist, und ein zweiter Partner des bioaffinen Bindepaares (P2) in freier Form vorlegt und der Verschluss über eine Wechselwirkung P1-P2-P1 erfolgt.

7. Verfahren nach einem der vorhergehenden Ansprüche,
**dadurch gekennzeichnet,**
**dass** die Ghosts von Gram-negativen Bakterien stammen.

8. Verfahren nach einem der vorhergehenden Ansprüche,
**dadurch gekennzeichnet,**
**dass** die Ghosts von rekombinanten Bakterien mit heterologen Membranpolypeptiden stammen.

9. Verfahren nach einem der vorhergehenden Ansprüche,
**dadurch gekennzeichnet,**
**dass** man als Trägermaterial-Lipidvesikel verwendet.

10. Verfahren nach Anspruch 9,
**dadurch gekennzeichnet,**
**dass** als Lipidvesikel Vesikel von homogenisierten Zellen, insbesondere Bakterienzellen, Liposomen oder membranumhüllte Viren verwendet werden.

11. Verfahren nach Anspruch 9 oder 10,
weiterhin umfassend eine zumindest teilweise Fusion der Membran der Bakterienghosts und der Membran der Lipidvesikel.

12. Verfahren nach einem der vorhergehenden Ansprüche,
**dadurch gekennzeichnet**,
weiterhin umfassend das Verpacken von Wirkstoffen in die Bakterienghosts.

13. Verfahren nach Anspruch 12,
**dadurch gekennzeichnet,**
**dass** die Wirkstoffe ausgewählt werden aus genetischem Material, Zellkomponenten, Substanzen, Markierungssubstanzen, landwirtschftlich wirksamen Substanzen, Farbstoffen und Kombinationen davon.

14. Verschlossener Bakterienghost, erhältlich durch das Verfahren nach einem der Ansprüche 1 bis 13, wobei der Verschluss über spezifische Wechselwirkungen zwischen Partnern eines bioaffinen Bindepaares vermittelt ist.

15. Verschlossener Bakterienghost nach Anspruch 14,
**dadurch gekennzeichnet,**
**dass** er eine zumindest teilweise intakte Membran enthält.

16. Verschlossener Bakterienghost nach Anspruch 14 oder 15,
**dadurch gekennzeichnet,**
**dass** er mindestens einen verkapselten Wirkstoff enthält.

17. Verschlossene Bakterienghosts nach einem der Ansprüche 14 bis 16 zur Verwendung in der Medizin.

18. Verwendung von verschlossenen Bakterienghosts nach einem der Ansprüche 14 bis 16 im Agrarbereich.

19. Verwendung von verschlossenen Bakterienghosts nach einem der Ansprüche 14 bis 16 in der Biotechnologie.

## Claims

1. A method for producing sealed bacterial ghosts, comprising bringing bacterial ghosts into contact with excipients under conditions in which the bacterial ghosts are sealed,
**characterised in that**
fusion is mediated by means of specific interactions between the partners of a bioaffinity binding pair which are anchored on the ghosts and/or the excipients.

2. A method according to claim 1,
**characterised in that**
the partners of the bioaffinity binding pair are selected from the group consisting of biotin or biotin analogues/streptavidin or avidin, hapten/antibody or antibody fragments, saccharide/lectin and ligand/receptor.

3. A method according to claim 2,
**characterised in that**
biotin/streptavidin is used as the bioaffinity binding pair.

4. A method according to any one of claims 1 to 3,
**characterised in that**
at least one partner of the bioaffinity binding pair is immobilised on the membrane of the bacterial ghosts and on the excipient.

5. A method according to claim 4,
**characterised in that**
a first partner of the bioaffinity binding pair (P1) is immobilised on the membrane of the bacterial ghosts and a second partner of the bioaffinity binding pair (P2) is immobilised on the excipient, and sealing is effected by a P1-P2 interaction.

6. A method according to claim 4,
**characterised in that**
a first partner of the bioaffinity binding pair (P1) is immobilised on the membrane of the bacterial ghosts and on the excipient, and a second partner of the bioaffinity binding pair (P2) is present in free form and sealing is effected by a P1-P2-P1 interaction.

7. A method according to any one of the preceding claims,
**characterised in that**
the ghosts originate from Gram-negative bacteria.

8. A method according to any one of the preceding claims,
**characterised in that**
the ghosts originate from recombinant bacteria with heterologous membrane polypeptides.

9. A method according to any one of the preceding claims,
**characterised in that**
lipid vesicles are used as the excipient.

10. A method according to claim 9,
**characterised in that**
vesicles of homogenised cells, in particular bacterial cells, liposomes or membrane-enveloped viruses are used as the lipid vesicles.

11. A method according to claim 9 or claim 10,
furthermore comprising at least partial fusion of the membrane of the bacterial ghosts and the membrane of the lipid vesicles.

12. A method according to any one of the preceding claims,
**characterised in that** [sic]
furthermore comprising packaging active ingredients in the bacterial ghosts.

13. A method according to claim 12,
**characterised in that**
the active ingredients are selected from genetic material, cell components, substances, labelling substances, agriculturally active substances, dyes and combinations thereof.

14. A sealed bacterial ghost, obtainable by the method according to any one of claims 1 to 13, wherein sealing is mediated by means of specific interactions between partners of a bioaffinity binding pair.

15. A sealed bacterial ghost according to claim 14,
**characterised in that**
it contains at least one partially intact membrane.

16. A sealed bacterial ghost according to claim 14 or claim 15,
**characterised in that**
it contains at least one encapsulated active ingredient.

17. Sealed bacterial ghosts according to any one of claims 14 to 16 for use in medicine.

18. Use of sealed bacterial ghosts according to any one of claims 14 to 16 in the agricultural sector.

19. Use of sealed bacterial ghosts according to any one of claims 14 to 16 in biotechnology.

## Revendications

1. Procédé de production de bactéries fantômes bloquées, comprenant la mise en contact des bactéries fantômes avec des matériaux support dans des conditions dans lesquelles un blocage des bactéries fantômes s'effectue,
**caractérisé en ce que**
la fusion est induite par le biais d'interactions spécifiques entre les partenaires d'un couple de liaison à affinité biologique qui sont ancrés sur les fantômes et/ou les matériaux support.

2. Procédé selon la revendication 1,
**caractérisé en ce que**
les partenaires du couple de liaison à affinité biologique sont choisis dans le groupe comprenant la biotine ou des analogues de biotine/streptavidine ou l'avidine, des haptènes/anticorps ou des fragments d'anticorps, un saccharide/lectine et un ligand/récepteur.

3. Procédé selon la revendication 2,
**caractérisé en ce que**
l'on utilise comme couple de liaison à affinité biologique, la biotine/streptavidine.

4. Procédé selon l'une des revendications 1 à 3,
**caractérisé en ce**
**qu'**au moins un partenaire du couple de liaison à affinité biologique est immobilisé sur la membrane des bactéries fantômes et sur le matériau support.

5. Procédé selon la revendication 4,
**caractérisé en ce**
**qu'**un premier partenaire du couple de liaison à affinité biologique (P1) est immobilisé sur la membrane des bactéries fantômes et un deuxième partenaire du couple de liaison à affinité biologique (P2) est immobilisé sur le matériau support et le blocage s'effectue par une interaction P1-P2.

6. Procédé selon la revendication 4,
**caractérisé en ce**
**qu'**un premier partenaire du couple de liaison à affinité biologique (P1) est immobilisé sur la membrane des bactéries fantômes et du matériau support et un deuxième partenaire du couple de liaison à affinité biologique (P2) se présente sous forme libre et le blocage s'effectue par une interaction P1-P2-P1.

7. Procédé selon l'une des revendications précédentes,
**caractérisé en ce**
**que** les fantômes proviennent de bactéries à gram négatif.

8. Procédé selon l'une des revendications précédentes,
**caractérisé en ce**
**que** les fantômes proviennent de bactéries recombinantes avec des polypeptides membranaires hétérologues.

9. Procédé selon l'une des revendications précédentes,
**caractérisé en ce**
**que** l'on utilise comme matériau support, des vésicules lipidiques.

10. Procédé selon la revendication 9,
**caractérisé en ce**
**que** l'on utilise comme vésicules lipidiques, des vésicules de cellules homogénéisées, en particulier des cellules bactériennes, des liposomes ou des virus entourés d'une membrane.

11. Procédé selon la revendication 9 ou 10,
comprenant en outre au moins une fusion partielle de la membrane des bactéries fantômes et de la membrane des vésicules lipidiques.

12. Procédé selon l'une des revendications précédentes,
**caractérisé en ce**
**qu'**il comprend en outre l'empaquetage de principes actifs dans les bactéries fantômes.

13. Procédé selon la revendication 12,
**caractérisé en ce**
**que** les principes actifs sont choisis parmi un matériau génétique, des composants cellulaires, des substances, des substances de marquage, des substances efficaces sur le plan agricole, des colorants et des combinaisons de ceux-ci.

14. Bactérie fantôme bloquée, pouvant être obtenue par le procédé selon l'une des revendications 1 à 13, le blocage étant induit par le biais d'interactions spécifiques entre les partenaires d'un couple de liaison à affinité biologique.

15. Bactérie fantôme bloquée selon la revendication 14,
**caractérisée en ce**
**qu'**elle contient une membrane au moins partiellement intacte.

16. Bactérie fantôme bloquée selon la revendication 14 ou 15,
**caractérisée en ce**
**qu'**elle contient au moins un principe actif encapsulé.

17. Bactéries fantômes bloquées selon l'une des revendications 14 à 16, pour leur utilisation en médecine.

18. Utilisation de bactéries fantômes bloquées selon l'une des revendications 14 à 16, dans le domaine agricole.

19. Utilisation de bactéries fantômes bloquées selon l'une des revendications 14 à 16, en biotechnologie.
